# EUROPEAN PATENT SPECIFICATION

(11) **EP 2 135 634 B1**
(45) Date of publication and mention of the grant of the patent: **19.10.2011**
(21) Application number: 09162884.2
(22) Date of filing: 16.06.2009
(51) Int. Cl.: A61M 25/01

(54) **Bi-Directional Asymmetric Steerable Sheath**
Bidirektionale asymmetrische lenkbare Ummantelung
Gaine orientable asymétrique bidirectionnelle

(30) Priority: 16.06.2008 US 61814 P
(43) Date of publication of application: 23.12.2009
(73) Proprietor: Greatbatch Ltd., Clarence, NY 14031 (US)
(72) Inventor: Farrell, Brian K., White Bear Lake, MN 55110 (US); Scheibe, Grant A., Loretto, MN 55357 (US); Pederson, Brian, East Bethel, MN 55092 (US)
(74) Representative: Duckett, Anthony Joseph

(56) References cited:
- EP-A- 1 679 094
- US-B1- 6 168 588

## Description

### BACKGROUND OF THE INVENTION

### Field of the Invention

The present invention generally relates to medical devices such as deflectable sheaths. More particularly, the present invention relates to a steerable sheath catheter for positioning in a desired orientation and location in the human body.

### SUMMARY OF THE INVENTION

Many current deflectable sheaths are designed to deflect in different directions to reach locations within the human body. These sheaths are composed of overlapping monolithic polymer layers that form continuous lumen(s). A wire mesh is typically placed between the monolithic polymer layers to provide added rigidity. Pull wire(s) are typically incorporated along the length of the sheath to provide a means of deflection.

Current sheaths however, have a limited deflection radius. When these sheaths are bent, the radius of curvature at the point of deflection is constant and symmetric about a deflection point. The sheath's arc of deflection is constant which therefore results in limited freedom of motion. This limitation substantially hinders the accessibility of the catheter to gain access to the desired location within the human body. Therefore, what is needed is a deflectable sheath that overcomes the shortcomings of previous designs by allowing the radius of curvature at the point of deflection to change, i.e. allow for asymmetric curvature about a point of deflection. This would allow the physician to gain access more easily in the human body particularly in the diseased vasculature which has been constricted with blockages.

The present invention is an improved deflectable sheath catheter that is capable of deflecting over a wider range of curvatures, i.e. is capable of deflecting over an asymmetric or non symmetrical range of curvatures. The improvement is directed to the use of a combination of materials with multiple durometers or hardnesses that reside within the outer lumen of the sheath.

EP 1679094 A1 describes a flexible tubular body for a catheter, sheath or similar medical device, in which different segments of the body may be made of polymer materials having different durometer values.

In manufacturing the catheter, a step or void is created in the region of intended deflection in the outer lumen layer. The step is then filled with a polymer(s) consisting of differing durometer(s). This creates a matrix of differing durometer polymers. Single or multiple steps can be made in the distal region of the outer lumen. These steps are also filled with a polymer(s) of differing durometer(s) to create a matrix of materials.

US 6,168,588 B1 describes a catheter in which a first length of tubular material is joined to a second length of tubular material by a welded joint. The joint may be a step weld. The tubular materials may have different durometer values.

Conventional sheath catheters do not have a step region that incorporates a combination of differing durometer materials. Instead, they are composed of lumen layers with each lumen layer composed of a monolithic polymer with a single and continuous durometer from one end to the other. The integration of a step region of differing durometer materials within an individual lumen enables the sheath catheter to deflect over a much wider range of curvatures than that provided by conventional deflectable sheaths. This asymmetric deflection functionality results in a sheath that is capable of a wider range of motion than previous deflectable sheaths.

### BRIEF DESCRIPTION OF THE DRAWINGS

FIG. 1 is a perspective view of a bi-directional steerable sheath assembly according to the present invention.
FIG. 2 depicts the preferred composition of the different polymer regions with different durometers in the outer layer lumen of the distal portion.
FIGS. 3A to 3C depict the various annular extensions of the different polymer regions that comprise the distal portion of the outer lumen as they wrap around the sheath and adjoin at the step interface.
FIG. 4 depicts an alternate composition of the different polymer regions with different durometers in the outer layer lumen of the distal portion.
FIG. 5 depicts an alternate composition of the different polymer regions with different durometers in the outer layer lumen of the distal portion.
FIG. 6 depicts examples of the different radius of curvatures capable of the sheath at the distal region.
FIG. 7 is a perspective view showing the addition of a ring 72 on the inner lumen.
FIG. 8 is a perspective view that shows the placement of the pull wires 80 and 82 along the inner lumen.
FIG. 9 is a perspective view that shows the method of attachment of the pull wires 80, 82 to the outer ring 72.
FIG. 10 is a perspective view that shows the placement of the wire mesh 140 over the sheath assembly of the inner lumen 70 and attached pull wires 80, 82.
FIG. 11 is a perspective view that shows the wire mesh 140 placed over the pull wires 80, 82 and inner lumen 70.
FIG. 12 is a perspective view that shows the placement of the second outer lumen 160 over the inner lumen, wire mesh and pull wire assembly depicted in FIG. 13.
FIG. 13 is a perspective view of the placement of the shrink wrap layer 170 over the sheath assembly comprising the wire mesh, pull wires, and inner and outer polymer lumens.
FIG. 14 is a perspective view of the entire sheath assembly comprising the inner and outer lumens, wire mesh, pull wires and shrink wrap.
FIG. 15 is a depiction of the sheath assembly being heat treated in a furnace 190.
FIG. 16 is a perspective view showing an exemplary deflectable sheath of the present invention having different durometer polymer regions that comprise the distal portion of the outer lumen.
FIG. 17 is an exploded cross-sectional view along line 17-17 of Fig. 16.
FIG. 18 is an exploded cross-sectional view along line 18-18 of Fig. 16.
FIG. 19 is an exploded cross sectional view along line 19-19 of Fig 16.

### DETAILED DESCRIPTION OF THE PREFERRED EMBODIMENTS

As used herein, the term "durometer" relates to the hardness of a material defined as a material's resistance to permanent indentation. In the hardness measurement of polymers, elastomers and rubbers according to the present invention, durometer is measured according to ASTM D2240 type A scale.

As used herein, a "step" is a transition from a first polymeric material to a second polymeric material where the first and second materials do not meet each other at an annular transition that forms a plane aligned generally perpendicular to a longitudinal axis of the sheath. An example is where the first polymeric material can range from 45° to 315° of the annular extent of the sheath member with the second polymeric material being the remainder of the annular extent along a cross-section aligned perpendicular to a longitudinal axis of the sheath. Multiple polymeric materials can be adjoined together around the catheter so as long as they together form a complete 360° annular extent around the sheath.

The present invention is a deflectable sheath 10 which is comprised of an elongated tubular structure that is flexible yet substantially non-compressible along its length. The deflectable sheath 10 extends from a deflectable distal portion 16 having a distal end 18, which is adapted to be disposed within a patient to a proximal portion 14. The sheath 10 is comprised of an outer tubular lumen member 160 formed of a polymeric material, such as of PEBAX. An inner tubular member 70 composed of a polymeric material, such as PTFE forms the inner lumen of the sheath. The PTFE inner lumen provides the sheath 10 with sufficient lubricity so that medical instruments, devices, and the like, slide through the sheath 10 with a minimal amount of force. A wire mesh 140 and pull wires 80 and 82 both formed of stainless steel, reside between the two lumen layers 70 and 160. A handle assembly 12, in turn, provides for selective deflection of a distal portion 16 of the sheath 10 into anyone of a number of disparate orientations, as will be further described in detail herein below. However, it is the incorporation of multiple durometer polymeric materials in the distal portion 16 of the outer lumen 160 that creates the extended asymmetric deflection of the sheath catheter as described in more detail below.

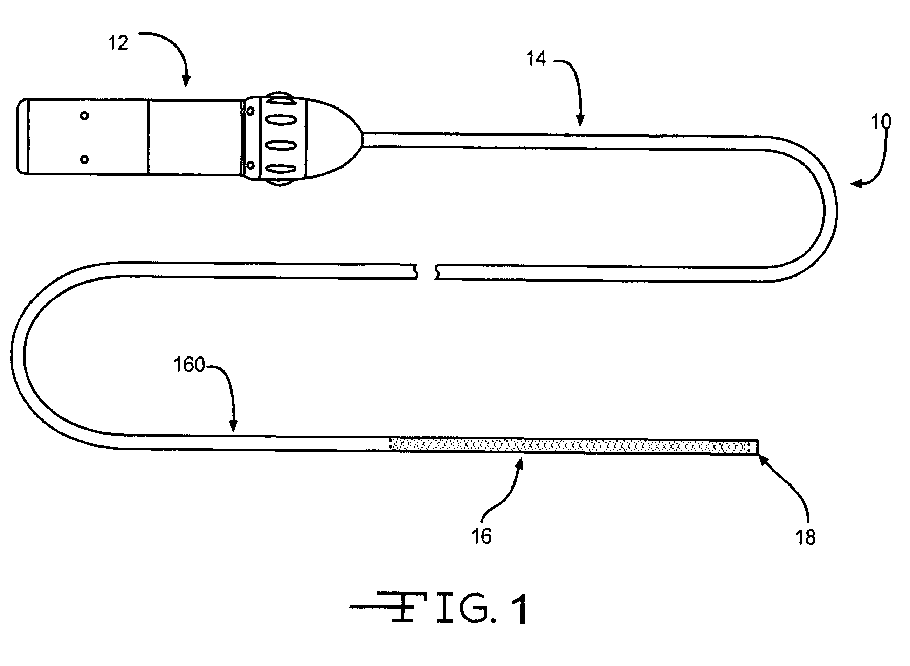

FIG. 1 illustrates a bi-directional asymmetric sheath assembly 10 according to the present invention. The deflectable sheath 10 has a length extending from a proximal portion 14 supported by the handle assembly 12 to a distal portion 16 and distal end 18. The distal portion 16, a section within the outer lumen 160, in turn is composed of at least two distal regions that are contiguous with each other at a step.

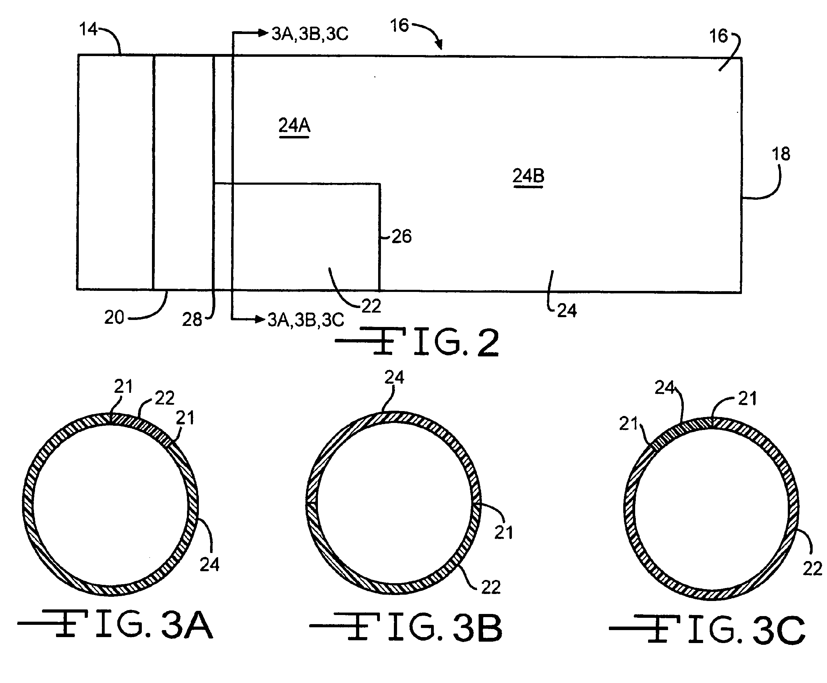

For example, FIG. 2 illustrates the distal portion 16 of the sheath 10 comprising a first distal region 20 composed of a polymeric material of a different durometer than the proximal portion 14. Preferably the first distal region 20 is composed of PEBAX of a 65 durometer that extends annularly about 360° around the sheath. The first distal region 20 meets a second distal region 22 composed of a polymeric material of a different durometer than the first region 20, such as 55 durometer PEBAX. The first distal region 20 meets the second distal region 22 and third distal region 24 at an annular transition 28 that forms a plane aligned generally perpendicular to a longitudinal axis of the sheath. The second distal region 22 extends annularly about 180° around the sheath and meets a third distal region 24 at a step 26. The third distal region 24 is composed of yet another polymeric material of a different durometer than the first and second distal regions 20 and 22, such as 35 durometer PEBAX. The third distal region 24 has a proximal portion 24A that extends annularly about 180° around the sheath and a distal portion 24B that extends to the end 18 of the distal portion 16 of the sheath 10.

In the exemplary construction shown in Fig. 2, materials with three different and distinct durometers compose the outer layer lumen of the sheath 10. However a combination of as few as two or a multitude of three or more distinct distal regions of a multitude of geometric shapes could also be used as long as the adjacent distal regions are composed of adjoining polymeric materials of differing durometers. Preferably the durometer of the polymeric material from one region differs by at least 10 durometers from the adjacent region. In that respect, for the sake of clarity the various regions of different durometers in a contiguous relationship with each other will be designated "first distal region", "second distal region", "third distal region", etc. as they extend from a most proximal distal region to the end of the distal portion 16 of the sheath 10.

More particularly with respect to FIG. 2, the deflectable sheath 10 of the present invention comprises the distal portion 16 extending for a length of about two inches to as much as about thirty-five inches with a diameter between about 0.1 inches to about 3 inches. The distal portion 16 is comprised of the first distal region 20 having a cylindrical shape that meets a second distal region 22 at an annular transition 28. If desired, the first distal region 20 is the proximal portion 14 supported by handle 12. In that case, there is no "middle sheath portion". In any event, the second distal region 22 meets the third distal region 24 at a step 26. Step 26 as depicted in Fig. 2 shows a 90° transition of the second distal region 22 to the third distal region 24 where it adjoins together and assumes a completely cylindrical shape extending to a distal end 18 of the distal sheath portion 16. Although depicted as a 90° angle as shown in step 26, the step transition can assume a multitude of different angles such as 45° and 180° or be of a curved transition boundary.

In that respect, the polymeric materials can have a wide range of annular extents, as long as they combine to have an annular extent of 360°. For example, the cross-section designated by line 3A-3A of FIG. 2 shows an embodiment where polymeric material 22 extends about 45° around the annular extent of the sheath while polymeric material 24 is the remainder of about 315°. In FIG. 2, cross-sectional line 3B-3B shows an embodiment where both materials 22 and 24 extend about 180° around the annular extent of the sheath. In FIG. 2, cross-sectional line 3C-3C shows an embodiment where polymeric material 22 extends about 315° around the annular extent of the sheath while polymeric material 24 is the remainder of about 45°. In each case, delineation between the respective materials 22 and 24 is designated by the abrupt transition line 21.

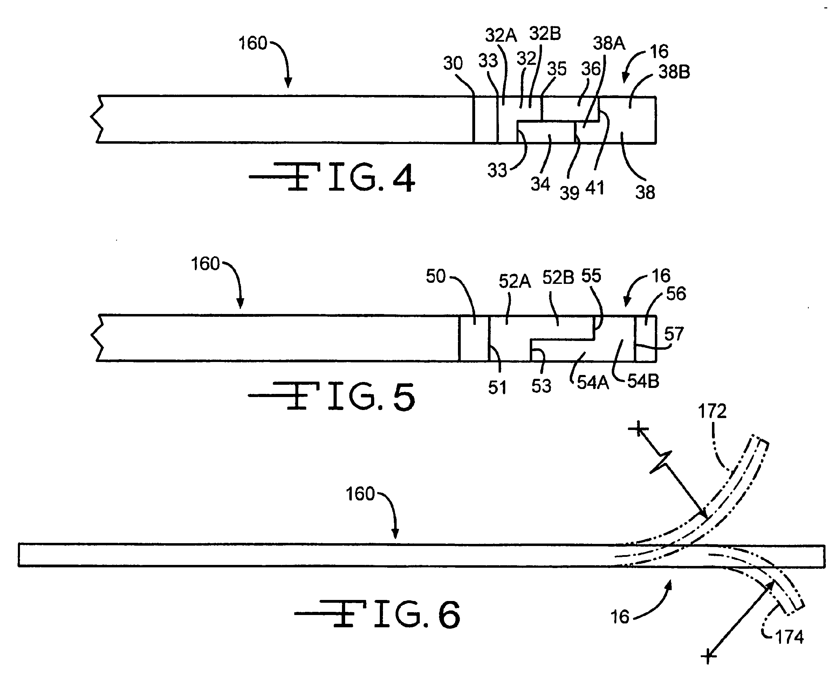

As further shown in FIG. 4, an alternate embodiment of the present deflectable sheath invention comprises a first distal region 30 of a durometer polymeric material having a cylindrical shape extending 360°. The first distal region 30 extends to and meets with a second distal region 32 of a polymeric material. The first distal region 30 can be composed of a PEBAX polymer of a durometer ranging from about 80 to about 65. The first distal region 30 meets the second distal region 32 at an annular transition at an annular transition that forms a plane aligned generally perpendicular to a longitudinal axis of the sheath 31. The second distal region 32 is comprised of a proximal portion 32A having a cylindrical shape extending 360° to a distal portion 32B extending somewhat less than that, for example 180°.

The second distal region 32 and a third distal region 34 are each of different durometer polymeric materials than that of the first distal region 30. The proximal portion 32A of the second distal region 32 extends to a step 33 where it meets the third distal region 34 having a cylindrical shape extending 180°about the periphery of the sheath.

The distal portion 32B of the second region 32 and the third distal region 34 in turn meet a fourth distal region 36 at a transition 37. The forth distal region 36 extends about 180° around the periphery of the sheath as a complementary portion to the distal portion 32B of the second region 32 and the third distal region 34.

The third distal region 34 in turn meets the proximal portion 38A of a fifth distal region 38 at a step 39. In turn, the distal portion 38B of the fifth distal region 38 meets the fourth distal region 36 at a step 41. Both distal regions 36 and 38 are of a different durometer. The proximal portion 38A of the fifth distal region 38 extends annularly about 180° around the sheath until it transitions into the distal portion 38B which has a cylindrical shape extending 360° to the distal end thereof. The fourth distal region 36 can be of a polymeric material having a durometer that is the same or different than that of the first and second distal regions 30 and 32. The fifth distal portion 38 meets a sixth distal region 40 at an annular transition that forms a plane aligned generally perpendicular to a longitudinal axis of the sheath, which in turn extends to the end 18 of the sheath 10.

In another embodiment, the second distal region 32 can be composed of a polymeric material such as PEBAX with a durometer ranging from about 75 to about 60. The third distal region 34 can be composed of a polymeric material such as PEBAX with a durometer ranging from about 70 to about 55. The fourth distal region 36 can be composed of a polymeric material such as PEBAX with a durometer ranging from about 65 to about 45, the fifth distal region 38 can be composed of a polymeric material such as PEBAX with a durometer ranging from about 55 to about 35 and the sixth distal region 40 having a durometer of from about 60 to about 50. The first distal region and the third or fourth distal regions 30, 34 or 36 can be of the same durometer material as long as adjoining distal regions are not of the same durometer.

Preferably, the durometer parameter decreases as the various polymeric materials extend to the distal end 18 of the sheath. However, that is not an absolute. In some designs, it may be desired to have a first polymeric material of a first durometer meeting a second polymeric material of a second durometer that in turn meets a third polymeric material of a third durometer. The third durometer can be less than both the first and second polymers or it can be less than one of them, but greater than the other.

FIG. 5 shows another alternate embodiment of the deflectable sheath invention. This alternate embodiment comprises a first distal region 50 which extends annularly about 360° about the sheath and meets the first portion 52A of the second distal region 52. Distal region 52 consists of a polymeric material of a different durometer than distal region 50 and extends annular about 180° about the sheath. Distal portion 52A extends to distal portion 52B of distal region 52. The distal portion 52A meets distal portion 54A, a distal portion of the third distal region 54, at step 53. Distal portion 52B meets distal portion 54B, an extension of distal portion 54A at step 55. A fourth distal region 56 which extends annularly about 360° about the sheath, meets distal portion 54B at an annular transition 57. Distal region 56 extends to the end of the sheath 18. Durometers of the polymeric material within the different distal regions can range from about 75 to about 25 with each adjacent distal region having a different durometer.

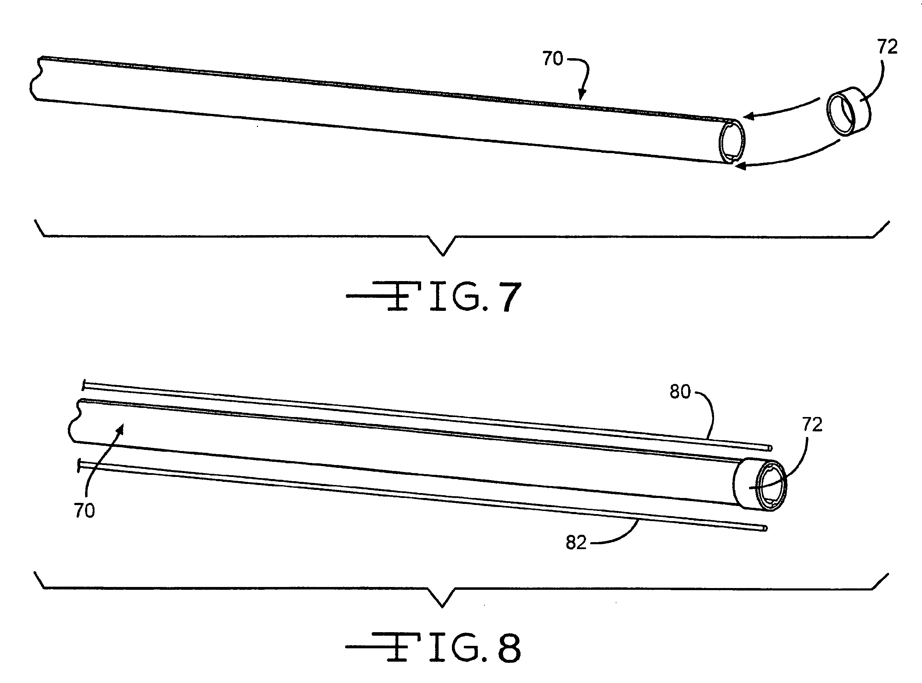

FIG. 8 shows the resulting extended asymmetrical deflection range of motion. With the added distal regions that are adjoined at annular transitions and interface steps of differing durometer polymeric materials, resulting in the asymmetric curvature of the distal portion 16 to 315° and more or as little as 45°. The deflectable sheath can bend at different or asymmetric angles about the deflection point; the larger angle of 315° is shown by numerical designation 172 while the smaller curvature of as little as 45° is shown by numerical designation 174.

As illustrated in FIG. 7, the manufacturing process of the deflectable sheath begins with an inner lumen 70 which consists of a monolithic PTFE material of a constant durometer. The lumen 70 is sized and shaped to receive, for example, instruments, fluids, media and the like. The length of this lumen is about twelve inches to about seventy inches long with a diameter of about 0.10 inches to about 1 inch.

A support ring 72 as shown in FIG. 7 is placed over the distal end of the inner PTFE lumen 70 and forms a tight fit. The distal support ring 72 is preferably made of stainless steel. The ring 72 can also be made of a different rigid material including but is not limited to, a rigid polymeric material, ceramic, titanium, copper, gold, silver, platinum, palladium, NITINOL^{®}, or other metal alloy. The purpose of the support ring 72 is to provide stability to the distal end as well as provide a support for attachment of the pull wire 80, 82.

Next pull wires 80 and 82 depicted in FIG. 8 are placed at opposing sides of the inner lumen. For maximum deflection the push/pull wires should be placed 90° from the complementary distal region. In other words, to provide maximum deflection, the opposing pull wires need to placed so that each of them lays across different durometer distal regions. Preferably the pull wires should be placed opposing each other and 90° from the transition steps of the previously described distal regions of varying durometers to create maximum deflection. These pull wires provide mechanical support to the sheath as well as a means for the operator to push and pull and consequently bend the catheter's distal region. The pull wires 80 and 82 extend from the support ring 72 at the distal end to the handle 12 where they connect to mechanisms for providing tension and compression to consequently deflect the distal portion 16 of the sheath 10 in one direction or another as previously described with respect to Fig. 6. Such push/pull mechanisms are well known by those skilled in the art and do not necessarily form a differentiating aspect of the present invention. The push/pull wires 80 and 82 are made of stainless steel material. However other materials including but not limited to copper, titanium, gold, silver, platinum, palladium, NITINOL^{®}, or flexible polymers and textile materials such as VECTRAN^{®} or Spectra can also be used.

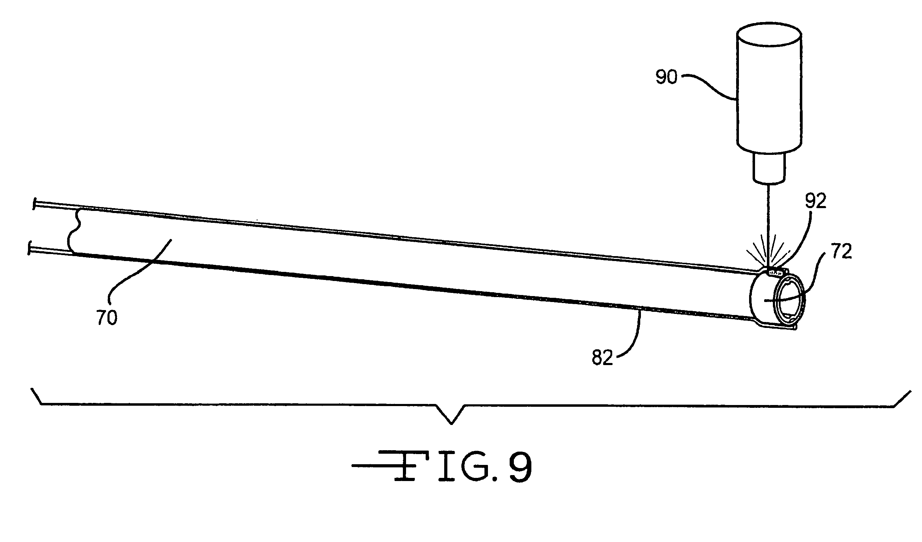

Push/pull wires 80 and 82, are then affixed to the distal support ring 72 by means of welding 90 such as laser or resistance welding 90 as depicted in FIG. 9. Alternate means of fixation include, but not limited to, chemical bonding, brazing, and soldering. The resulting attachment bond created either through welding, brazing, soldering or other means is depicted as 92.

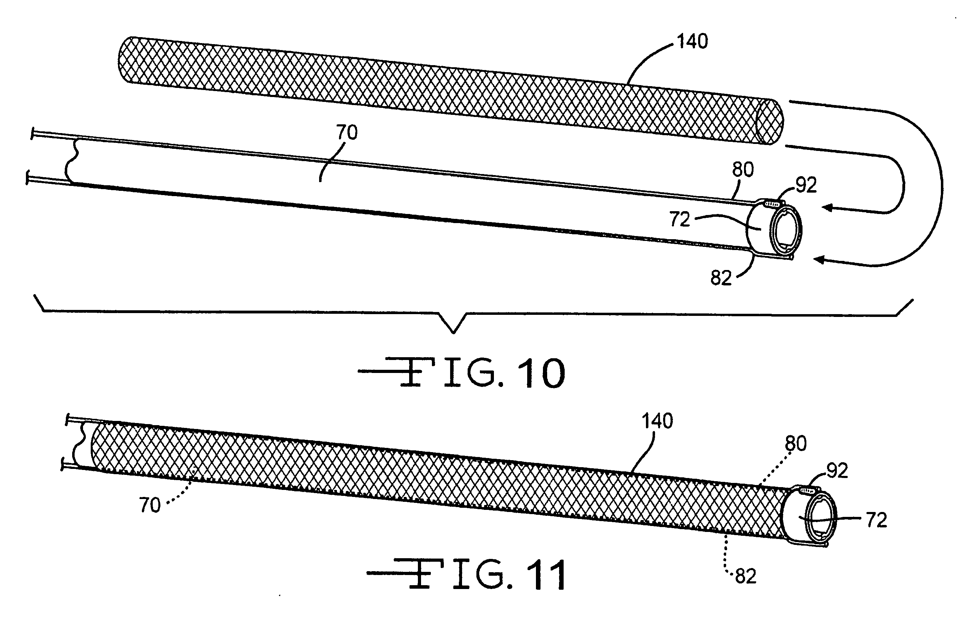

Following attachment of the pull wires 80, 82 as shown in FIG. 9, a stainless steel wire mesh 140 is placed over the assembly as shown in FIG. 10. The wire mesh 140 is pulled over the inner lumen/pull wire assembly and forms a tight fit over the inner PTFE lumen 70 and opposing pull wires 80 and 82 as shown in FIG. 11. The steel wire mesh 140 provides additional mechanical support to the sheath. The addition of the wire mesh 140 is also known to those skilled in the art and the addition of the wire mesh does not form a differentiating aspect of the present invention. Preferably the wire mesh is composed of stainless steel. Alternate wire braid mesh materials may include NITINOL^{®,} titanium, copper, nickel, gold, silver, palladium, platinum, ceramic or rigid polymer.

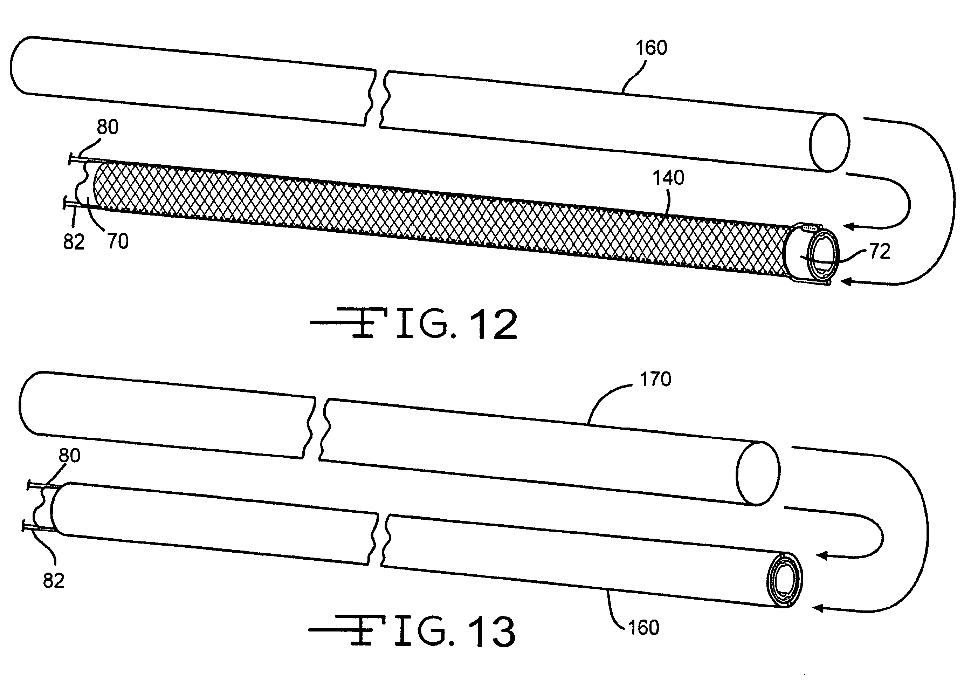

Following the addition of the stainless steel wire mesh 140 as shown in FIG. 11, a second polymeric lumen of PEBAX is placed over the sheath assembly as shown in FIG. 12. The lumen is composed of a high durometer polymeric material, preferably of PEBAX, having a durometer from about 50 to about 150. The preferred durometer of the proximal region is between 70 and 75. The length of the outer lumen corresponds to that of the inner lumen and can be about twelve inches to about seventy inches long with a diameter of about 0.10 inches to about 3 inches.

A step or steps are cut in the area of intended deflection in the distal portion 16 of the outer lumen material typically by splitting the outer lumen. The removal of the material from the outer lumen creates the space for the different durometer polymeric material. The step or steps are then filled with a geometrically matching piece of material of differing durometer as shown in FIGs. 2, 4, and 5. This results in the creation of the different distal regions that comprise the distal portion of the outer lumen of the sheath. The filling material is PEBAX with a durometer that is typically less than 75. Other polymeric materials could also be used provided that the alternate polymeric material has a different durometer and readily fuses together with the outer lumen layer 160 material.

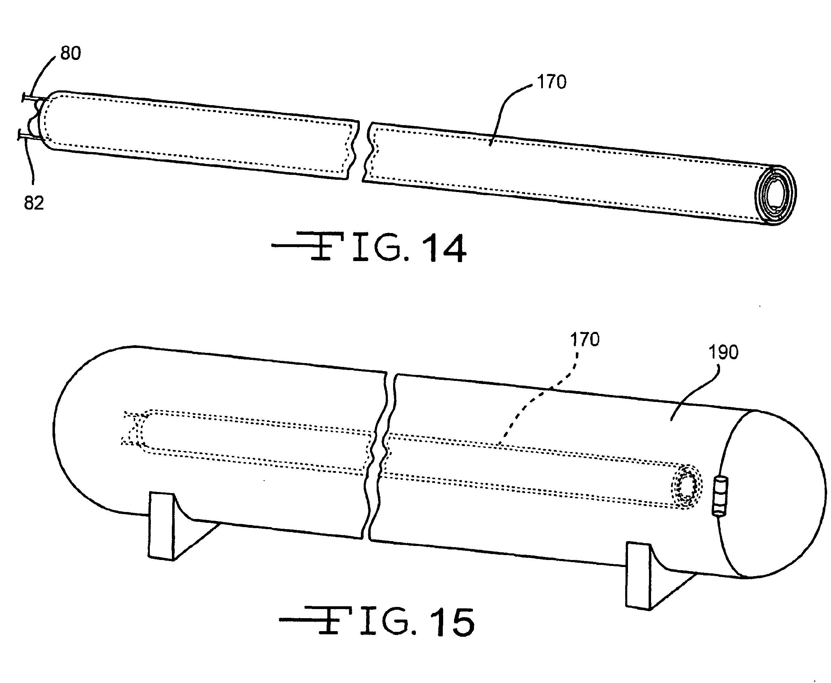

The entire assembly of the PTFE inner lumen 70, push/pull wires 80 and 82, wire mesh 140 and outer lumen 160 is then encased in a shrink wrap material 170 as shown in Fig. 13. The sheath assembly with the shrink wrap material 170 ready for heat treating is shown in FIG. 14. The assembly is then heat treated in a furnace 190 as shown in FIG. 15 at a preferred temperature of between about 350°F to about 450°F for about 5 to about 10 minutes in ambient atmosphere and pressure to create the final assembly. After heat treating, the remaining shrink wrap material 170 is removed from the surface of the sheath 10.

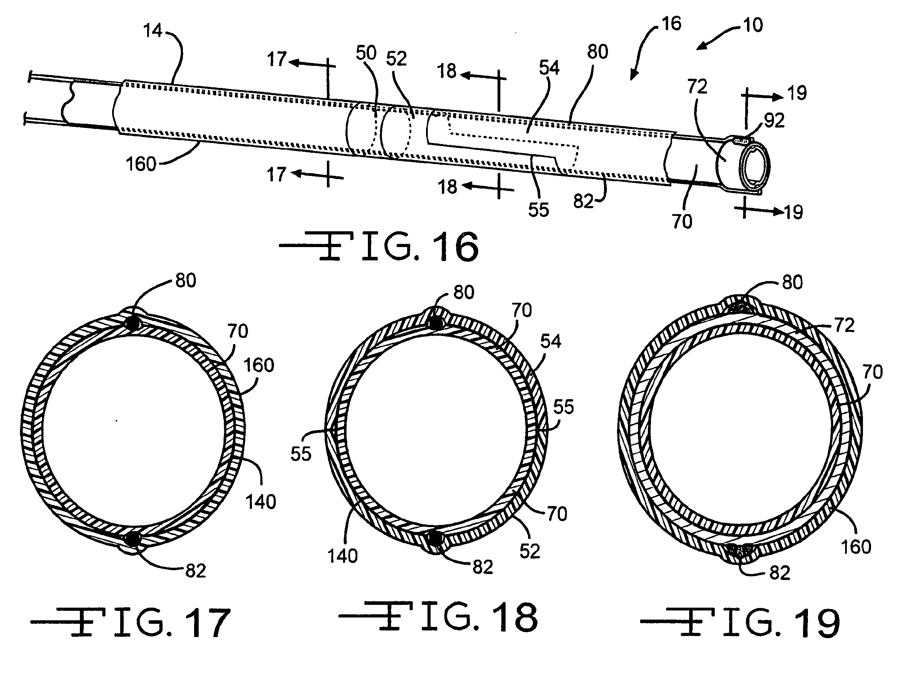

FIG. 16 shows an exemplary embodiment of a finished bi-directional asymmetric steerable sheath 10 according to the present invention. The illustration depicts the sheath 10 from the proximal region 14 through the distal portion 16 and shows the inner PTFE lumen 70, distal support ring 72, wire mesh 140 and push/pull wires 80 and 82 which attachment weld 92 also included in the illustration are distal regions 50, 52, and 54 similar to that which is depicted in FIG5. The first distal region 50 extends annularly 360 around the sheath 10. Distal region 50 adjoins distal region 52 which then adjoins distal region 54 at annular transition 55 which then extends to the end of the sheath. FIG. 17 illustrates the cross section at point 17-17 which is prior to the distal portion 16 section which shows the inner lumen 70, monolithic outer lumen 160, wire mesh 140 and push/pull wires 80 and 82. FIG 18 illustrates cross section 18-18 which depicts the outer lumen distal regions of 54 and 52, annular transition 55, the inner lumen 70, wire mesh 140 and push/pull wires 80 and 82. Finally FIG. 19 depicts the cross section 19-19 of the above sheath assembly at the distal end. The cross section shown in FIG. 19 consists of the inner PTFE lumen layer 70, distal end support ring 72, and pull wires 80 and 82 which are welded to the opposing sides.

Thus, it can be seen that the present invention provides a physician with a sheath assembly 10 that is capable of readily deflecting the distal portion 16 in any one of a myriad of direction, both upwardly and downwardly with respect to a longitudinal axis thereof as shown in Fig. 16. This provides the physician with a great degree of flexibility in maneuvering the distal end 16 of the sheath 10 for performing a medical procedure inside the vasculature of a patient.

It is appreciated that various modifications to the inventive concepts described herein may be apparent to those of ordinary skill in the art without departing from the scope of the present invention as defined by the appended claims.

## Claims

1. A deflectable sheath, which comprises:
a) a tubular sheath comprised of at least a first tubular member having a length extending from a proximal sheath portion to a deflectable distal sheath portion, the tubular sheath providing a delivery lumen extending to a distal end of the distal sheath portion;
b) a handle supporting the proximal sheath portion;
c) first and second push/pull wires disposed diametrically opposite each other and extending from the handle along the sheath to the deflectable distal sheath portion; and
d) wherein at least the first tubular member of the sheath is comprised of at least a first polymeric material extending from the proximal sheath portion part way along the sheath length to a second polymeric material extending to the distal end thereof, the first and second polymeric materials being composed of differing durometer materials; **characterized in that**
the first and second polymeric materials meet each other at a step transition, wherein at the step transition, the first polymeric material has a first annular extent of about 180° around the circumference of the tubular sheath along a cross-section aligned perpendicular to a longitudinal axis of the sheath and the second polymeric material has a second annular extent of about 180° around the remainder of the circumference of the tubular sheath, and wherein the push/pull wires are offset about 90° from the step transition where the first polymeric material meets the second polymeric material.

2. The deflectable sheath of claim 1 wherein the first polymeric material is of a higher durometer than the second polymeric material.

3. The deflectable sheath of claim 1 or claim 2 wherein the first polymeric material ranges from 80 durometer to 45 and the second polymeric material ranges from 55 durometer to 25 durometer.

4. The deflectable sheath of any of claims 1 to 3 wherein the first polymeric material has a proximal first polymeric portion having a first annular extent of 360° around the circumference of the tubular sheath and a distal first polymeric portion having a second annular extent of about 180° around the circumference of the tubular sheath along a cross-section aligned perpendicular to a longitudinal axis of the sheath and wherein the second polymeric material has a proximal second polymeric portion having a first annular extent of about 180° around the circumference of the tubular sheath at the cross-section and a distal second polymeric portion having a second annular extent of 360° around the circumference of the tubular sheath.

5. The deflectable sheath of claim 4 wherein the distal second polymeric portion having the second annular extent of 360° around the circumference of the tubular sheath extends to the distal end of the sheath.

6. The deflectable sheath of any of claims 1 to 5 further including a third polymeric material disposed between the first polymeric material and at least a portion of the second polymeric material.

7. The deflectable sheath of claim 6 wherein the first polymeric material meets the second and third polymeric materials at an annular transition that forms a plane aligned generally perpendicular to a longitudinal axis of the sheath and the third polymeric material has a durometer that is either greater than or less than that of the second polymeric material.

8. The deflectable sheath of claim 6 wherein the second and third polymeric materials meet each other at a step transition.

9. The deflectable sheath of claim 6 wherein the third polymeric material has a first annular extent of from 45° to 315° around the circumference of the tubular sheath along a cross-section aligned perpendicular to a longitudinal axis of the sheath and the second polymeric material has a second annular extent of from 315° to 45° around the remainder of the circumference of the tubular sheath where the second and third polymeric material meet the first polymeric material at the cross-section.

10. The deflectable sheath of claim 6 wherein the second polymeric material has a proximal second polymeric portion having an annular extent of from 45° to 315° around the circumference of the tubular sheath along a cross-section aligned perpendicular to a longitudinal axis of the sheath and a distal second polymeric portion having an annular extent of 360° around the circumference of the tubular sheath.

11. The deflectable sheath of any of claims 6 to 10 further including a fourth polymeric material extending from the distal second polymeric portion to the distal end of the sheath and the fourth polymeric material has a durometer that is either greater than or less than that of the third polymeric material.

12. The deflectable sheath of any of claims 1 to 11 wherein there is provided a second tubular member disposed inside the first tubular member and a wire mesh intermediate the first and second tubular members.

## Patentansprüche

1. Eine lenkbare Ummantelung, welche umfasst:
a) eine röhrenartige Ummantelung, welche wenigstens ein erstes röhrenartiges Element umfasst, welches eine Länge aufweist, welche sich von einem proximalen Ummantelungsabschnitt hin zu einem ablenkbaren distalen Ummantelungsabschnitt erstreckt, wobei die röhrenartige Ummantelung ein Abgabelumen, welches sich zu einem distalen Ende des distalen Ummantelungsabschnittes hin erstreckt, bereitstellt;
b) ein Griff, welcher den proximalen Ummantelungsabschnitt abstützt;
c) erste und zweite Druck-/Zugdrähte, welche diametral einander gegenüber liegend angeordnet sind und welche sich vom Griff entlang der Ummantelung hin zu dem ablenkbaren distalen Ummantelungsabschnitt erstrecken; und
d) wobei wenigstens das erste röhrenartige Element der Ummantelung wenigstens ein erstes kunststoffartiges Material umfasst, welches sich von dem proximalen Ummantelungsabschnitt teilweise entlang der Ummantelungslänge hin zu einem zweiten kunststoffartigen Material, welches sich zu dem distalen Ende hiervon erstreckt, erstreckt, wobei erste und zweite kunststoffartige Materialien aus unterschiedlichen Härtegradmaterialien zusammengesetzt sind
**dadurch gekennzeichnet,dass**
sich die ersten und zweiten kunststoffartigen Materialien an einem Stufenübergang treffen, wobei an diesem Stufenübergang das erste kunststoffartige Material einen ersten ringförmigen Bereich von etwa 180° um den Umfang der röhrenartigen Ummantelung entlang eines Querschnitts, welcher senkrecht zu der Längsachse der Ummantelung ausgerichtet ist, aufweist, und das zweite kunststoffartige Material einen zweiten ringförmigen Bereich von etwa 180° um den restlichen Umfang der röhrenartigen Ummantelung aufweist und wobei die Druck-/Zugdrähte um etwa 90° von dem Stufenübergang, wo das erste kunststoffartige Material das zweite kunststoffartige Material trifft, versetzt sind.

2. Lenkbare Ummantelung nach Anspruch 1, wobei das erste kunststoffartige Material einen höheren Härtegrad als das zweite kunststoffartige Material aufweist.

3. Lenkbare Ummantelung nach Anspruch 1 oder Anspruch 2, wobei das erste kunststoffartige Material im Bereich von 80 bis 45 Härtegrade und das zweite kunststoffartige Material im Bereich von 55 bis 25 Härtegrade liegt.

4. Lenkbare Ummantelung nach einem der Ansprüche 1 - 3, wobei das erste kunststoffartige Material einen proximalen ersten kunststoffartigen Abschnitt aufweist, welcher einen ersten ringförmigen Bereich von 360° um den Umfang der röhrenartigen Ummantelung aufweist, und einen distalen ersten kunststoffartigen Abschnitt, welcher einen zweiten ringförmigen Bereich von 180° um den Umfang der röhrenartigen Ummantelung entlang eines Querschnitts, welcher senkrecht zu der Längsachse der Ummantelung angeordnet ist, aufweist, und wobei das zweite kunststoffartige Material einen proximalen zweiten kunststoffartigen Abschnitt aufweist, welcher einen ersten ringförmigen Bereich von etwa 180° um den Umfang der röhrenartigen Ummantelung am Querschnitt aufweist, und einen distalen zweiten kunststoffartigen Abschnitt aufweist, welcher einen zweiten ringförmigen Bereich von 360° um den Umfang der röhrenartigen Ummantelung aufweist.

5. Lenkbare Ummantelung nach Anspruch 4, wobei sich der distale zweite kunststoffartige Abschnitt, welcher den zweiten ringförmigen Bereich von 360° um den Umfang der röhrenartigen Ummantelung aufweist, zum distalen Ende der Ummantelung hin erstreckt.

6. Lenkbare Ummantelung nach einem der Ansprüche 1 - 5 weiter beinhaltend ein drittes kunststoffartiges Material, welches zwischen dem ersten kunststoffartigen Material und wenigstens eines Teils des zweiten kunststoffartigen Materials angeordnet ist.

7. Lenkbare Ummantelung nach Anspruch 6, wobei das erste kunststoffartige Material die zweiten und dritten kunststoffartigen Materialien an einen ringförmigen Übergang trifft, welcher eine Ebene bildet, welche im allgemeinen senkrecht zu der Längsachse der Ummantelung ausgerichtet ist, und das dritte kunststoffartige Material einen Härtegrad aufweist, welcher entweder größer oder kleiner als der des zweiten kunststoffartigen Materials ist.

8. Lenkbare Ummantelung nach Anspruch 6, wobei die zweiten und dritten kunststoffartigen Materialien sich an einem Stufenübergang treffen.

9. Lenkbare Ummantelung nach Anspruch 6, wobei das dritte kunststoffartige Material einen ersten ringförmigen Bereich von 45° bis 315° um den Umfang der röhrenartigen Ummantelung entlang eines Querschnitts, welcher senkrecht zu der Längsachse der Ummantelung angeordnet ist, aufweist, und das zweite kunststoffartige Material einen zweiten ringförmigen Bereich von 315° bis 45° um den restlichen Umfang der röhrenartigen Ummantelung aufweist, wo das zweite und dritte kunststoffartige Material das erste kunststoffartige Material am Querschnitt treffen.

10. Lenkbare Ummantelung nach Anspruch 6, wobei das zweite kunststoffartige Material einen proximalen zweiten kunststoffartigen Abschnitt, welcher einen ringförmigen Bereich von 45° bis 315° um den Umfang der röhrenartigen Ummantelung entlang eines Querschnitts, welcher senkrecht zu der Längsachse der Ummantelung ausgerichtet ist, aufweist, und einen distalen zweiten kunststoffartigen Abschnitt aufweist, welcher einen ringförmigen Bereich von 360° um den Umfang der röhrenartigen Ummantelung aufweist.

11. Lenkbare Ummantelung nach einem der Ansprüche 6 - 10 weiter beinhaltend ein viertes kunststoffartiges Material, welches sich von dem distalen zweiten kunststoffartigen Abschnitt hin zu dem distalen Ende der Ummantelung erstreckt und das vierte kunststoffartige Material einen Härtegrad aufweist, welcher entweder größer oder kleiner als der des dritten kunststoffartigen Materials ist.

12. Lenkbare Ummantelung nach einem der Ansprüche 1 - 11, wobei ein zweites röhrenartiges Element, welches im Inneren des ersten röhrenartigen Elements angeordnet ist, und ein Drahtnetz bereitgestellt wird, welches zwischen dem ersten und zweiten röhrenartigen Element liegt/angeordnet ist.

## Revendications

1. Gaine orientable, comprenant :
a) une gaine tubulaire composée d'au moins un premier organe tubulaire ayant une longueur s'étendant d'une partie de gaine proximale à une partie de gaine distale orientable, la gaine tubulaire assurant un conduit de livraison s'étendant jusqu'à une extrémité distale de la partie de gaine distale ;
b) une poignée supportant la partie de gaine proximale ;
c) des premier et deuxième fils de poussée/tirage disposés diamétralement opposés l'un à l'autre et s'étendant de la poignée le long de la gaine jusqu'à la partie de gaine distale orientable ; et
d) dans laquelle au moins le premier organe tubulaire de la gaine se compose d'au moins un premier matériau polymérique s'étendant de la partie de gaine proximale sur la longueur de gaine jusqu'à un deuxième matériau polymérique s'étendant jusqu'à l'extrémité distale de celle-ci, les premier et deuxième matériaux polymériques étant composés de matériaux de dureté différente ;
**caractérisée en ce que**
les premier et deuxième matériaux polymériques se rencontrent à une transition en escalier, dans laquelle, à la transition en escalier, le premier matériau polymérique a une première étendue annulaire d'environ 180° autour de la circonférence de la gaine tubulaire le long d'une coupe transversale alignée perpendiculairement à un axe longitudinal de la gaine et le deuxième matériau polymérique a une deuxième étendue annulaire d'environ 180° autour du reste de la circonférence de la gaine tubulaire, et dans laquelle les fils de poussée/tirage sont décalés d'environ 90° par rapport à la transition en escalier à laquelle le premier matériau polymérique rencontre le deuxième matériau polymérique.

2. Gaine orientable selon la revendication 1, dans laquelle le premier matériau polymérique est d'une dureté supérieure à celle du deuxième matériau polymérique.

3. Gaine orientable selon la revendication 1 ou 2, dans laquelle le premier matériau polymérique a une dureté dans une plage de 80 à 45 et le deuxième matériau polymérique a une dureté dans une plage de 55 à 25.

4. Gaine orientable selon l'une quelconque des revendications 1 à 3, dans laquelle le premier matériau polymérique a une première partie polymérique proximale ayant une première étendue annulaire de 360° autour de la circonférence de la gaine tubulaire et une première partie polymérique distale ayant une deuxième étendue annulaire d'environ 180° autour de la circonférence de la gaine tubulaire le long d'une coupe transversale alignée perpendiculairement à un axe longitudinal de la gaine et dans laquelle le deuxième matériau polymérique a une deuxième partie polymérique proximale ayant une première étendue annulaire d'environ 180° autour de la circonférence de la gaine tubulaire à la coupe transversale et une deuxième partie polymérique distale ayant une deuxième étendue annulaire de 360° autour de la circonférence de la gaine tubulaire.

5. Gaine orientable selon la revendication 4, dans laquelle la deuxième partie polymérique distale ayant la deuxième étendue annulaire de 360° autour de la circonférence de la gaine tubulaire s'étend jusqu'à l'extrémité distale de la gaine.

6. Gaine orientable selon l'une quelconque des revendications 1 à 5, comprenant en outre un troisième matériau polymérique disposé entre le premier matériau polymérique et au moins une partie du deuxième matériau polymérique.

7. Gaine orientable selon la revendication 6, dans laquelle le premier matériau polymérique rencontre le deuxième matériau polymérique et le troisième matériau polymérique à une transition annulaire qui forme un plan aligné généralement perpendiculairement à un axe longitudinal de la gaine et le troisième matériau polymérique a une dureté qui est supérieure ou inférieure à celle du deuxième matériau polymérique.

8. Gaine orientable selon la revendication 6, dans laquelle les deuxième et troisième matériaux polymériques se rencontrent à une transition en escalier.

9. Gaine orientable selon la revendication 6, dans laquelle le troisième matériau polymérique a une première étendue annulaire de 45° à 315° autour de la circonférence de la gaine tubulaire le long d'une coupe transversale alignée perpendiculairement à un axe longitudinal de la gaine et le deuxième matériau polymérique a une deuxième étendue annulaire de 315° à 45° autour du reste de la circonférence de la gaine tubulaire où les deuxième et troisième matériaux polymériques rencontrent le premier matériau polymérique à la coupe transversale.

10. Gaine orientable selon la revendication 6, dans laquelle le deuxième matériau polymérique a une deuxième partie polymérique proximale ayant une étendue annulaire de 45° à 315° autour de la circonférence de la gaine tubulaire le long d'une coupe transversale alignée perpendiculairement à un axe longitudinal de la gaine et une deuxième partie polymérique distale ayant une étendue annulaire de 360° autour de la circonférence de la gaine tubulaire.

11. Gaine orientable selon l'une quelconque des revendications 6 à 10, comprenant en outre un quatrième matériau polymérique s'étendant de la deuxième partie polymérique distale à l'extrémité distale de la gaine et le quatrième matériau polymérique a une dureté qui est supérieure ou inférieure à celle du troisième matériau polymérique.

12. Gaine orientable selon l'une quelconque des revendications 1 à 11, dans laquelle un deuxième organe tubulaire est disposé à l'intérieur du premier organe tubulaire et une grille est disposée entre le premier organe tubulaire et le deuxième organe tubulaire.
